# EUROPEAN PATENT APPLICATION

(11) **EP 4 632 142 A1**
(43) Date of publication of application: **15.10.2025**
(21) Application number: 24169246.6
(22) Date of filing: 09.04.2024
(51) Int. Cl.: D06M 10/00, D06M 10/02, D06M 10/06, D06M 10/08, D06M 11/74, D06M 13/02, D06M 13/507, D06M 13/513, D06M 14/18, D06M 14/28, D06M 23/16

(54) **METHOD OF PRODUCING A FABRIC HAVING A HYDRO- AND OLEOPHOBIC CHARACTERISTICS**

(71) Applicant: Sefar AG, 9410 Heiden (CH)
(72) Inventor: HOSSAIN, Mohammad Mokbul, 9410 Heiden (CH); CHAKHARI, Karim, 9000 St. Gallen (CH); ELLENBERGER, Christoph, 9451 Kriessern (CH)
(74) Representative: Wunderlich & Heim Patentanwälte PartG mbB

(57) **Abstract**

The invention relates to a method of producing a fabric having a halogen free plasma coating, having a hydro- and oleophobic characteristics, wherein the method comprises: step DHF of depositing a plasma coating on the fabric by means of plasma polymerization of a halogen free precursor monomer by plasma-enhanced chemical vapor deposition method (PECVD), wherein the halogen free precursor monomer are organosilane, siloxane and/or hydrocarbon precursors, wherein the plasma-enhanced chemical vapor deposition is carried out as a low-pressure plasma processes under protective atmosphere, wherein the fabric comprises of a woven monofilament fabric of polymeric material having a filament diameter between 10 µm to 150 µm and a mesh opening between 5 µm and 200 µm.

## Description

The invention relates to a method of producing a fabric having a halogen free plasma coating, especially free of per- and polyfluorinated substances (PFAS), according to standard IEC 62321-3-2:2020, EN 14582:2016 and/or ASTM D7359:2018, having a hydro- and oleophobic characteristics. According to the inventions these characteristics can be evaluated for water, diiodomethane and hexadecane contact angle according to DIN 55660-2:2011-12 and for oil grade according to DIN EN ISO 14419:2010.

Further the invention also relates to a fabric comprising halogen free plasma coating, especially free of per- and polyfluorinated substances (PFAS), according to standard IEC 62321-3-2:2020, EN 14582:2016 and/or ASTM D7359:2018 formed thereon having a hydro- and oleophobic characteristics.

Anthropogenic organic compounds like per- and polyfluorinated alkyl substances are among substances of very high concern (SVHCs) and represent a large family which have been used in a variety of industries. The literature has reported the use of these compounds as processing additives and as surfactants since 1940s. These compounds, which have special properties including fire resistance and oil, stain, grease, and water repellency, have been used commonly in the production of non-stick cookware, specialized garments and textiles, stain repellents, metal plating, and firefighting foams. They are classified in two groups of PFAS; perfluoroalkyl sulfonic acids (PFSA) and perfluorocarboxylic acids (PFCA). These synthetic substances that do not occur naturally in the environment and PFAS including its related salts has been already detected in different types of aqueous environments with different concentration. This is not surprising at all since certain PFAS are persistent and bioaccumulative.

PFAS are increasingly detected as environmental pollutants and some are linked to negative effects on human health. Moreover they are very resistant to degradation once they occur because of the strength of C-F bond. In addition, it has been recorded that most PFAS are also easily transported in the environment covering long distances away from the source of their release. C8 based PFAS have already been listed as regulatory substances in the EU, and perfluorooctanesulfonic acid (PFOS) has been classified as persistent organic pollutants (POPs) in 2009.

Since 2015 it is even banned in several countries to formulate products which contains PFOS and a large amounts of PFOA. Even the use of C6 Fluorocarbon (FC) has resulted in global environmental contamination because it contains a huge amount of PFAS and traces of perfluorooctanoic acid (PFOA) respectively its salts. Thus the concern has been raised because of the persistence and potential for bioaccumulation of these substances. Consequently the REACH regulation (EU/784/2020) which is effective from 3 December 2020 has limited the PFOA threshold value to be under 25 ppb (parts per billion).

This has started a shift from longer chain FC (C8, C6) to ultrashort chain C3 to C1 fluorocarbon like per- and polyfluoroalkyl substances (PFAS). Even though these compounds in principle provide hydrophobic and oleophobic characteristics, it was realized that these short chain monomers and polymers still contain a fair quantity of fluorine in order to obtain comparable hydro- and oleophobic performance as compared to long chain FC. Although the effects of long-term exposure of PFAS containing substances to humans and environment are not fully known, a health hazard still exist also with short chain based coating.

Unless the necessary measures to restrict these very persistent compounds have been not taken, people, plants and animals may be increasingly exposed to hazard. It is estimated that around 4.4 million tons of PFASs would end up in the environment over the next 30 years unless action is taken.

Therefore, a new regulation process has started and the respective regulation draft was prepared by respective authorities and submitted to ECHA in January 2023. It aims to reduce PFAS emissions into the environment and make products and processes safer for people. This revolutionary proposal will not be focused on one chemical only, as implemented until now, but will concern all PFAS categories. The regulation proposal dossier (Annex XV of REACH) defined that, among others, the main application areas include the TULAC (textiles, upholstery, leather, apparel and carpets) areas. The proposal imposes a limit of 25 ppb for any PFAS, 250 ppb for the sum of PFAS, and 50 ppm for polymeric PFAS calculated as total fluorine.

While the restriction is progressing forward and its enforcement might be sooner, the demand on PFAS free hydro- and oleophobic coatings is increasing drastically. Silicon-based coatings, mainly comprising silanes and siloxanes, are considered safer alternatives for fabrics. Chemically organosilicons consist of Si atoms bonded to organic hydrocarbon groups. It comprises organosilanes (trimethyl silane etc.) and siloxans (hexamethyldisiloxane, tetramethylsilane etc.). It has been reported that Si-based films deposited by plasma are of interest for semi-conductor fabrication and also for flexible solar cells.

EP 3 101 17 0A1 teaches fluorine-free durable plasma nanocoating on fabric substrate by means of low pressure plasma polymerization. The coating should provide an adequate level of water repellency i.e. hydrophobicity to certain textiles. With regard to the durability, the coating applied to textile fabric subjected to repeated washing and an reasonable level of water repellency after reasonable number of washing cycles should be able to obtain.

WO 2022/171581 A1discloses a hollow cathode plasma polymerization process applied to fabric substrates to obtain a durable halogen-free, in particular fluorine-free, hydrophobic polymer coating.

However as for textiles plasma polymerized organosilicons have been deposited to ensure dielectric properties, thermal stability, scratch resistance as well as to adjust the wettability ie. hydrophobicity but so far no oleophobic properties were reported.

Siloxanes are widely used as precursor monomer for plasma processes to obtain fluorine-free plasma coatings. Plasma polymers from those precursors shows promising mechanical properties such as low internal stress, good adhesion and excellent hydrophobic barrier performance. Although the water resistance provided by plasma coating of e.g. TMDSO is promising, but it does not pose any oleophobic properties as also disclosed by EP 4 177 050 A1. Therefore, a general trend is to obtain a good hydro- and oleophobic coating for PFAS restriction reasons. Moreover, there is still demand for next generation super hydro- and oleophobic coating with high degree of repellency against oils and fats.

It is therefore the **object** of the invention to provide a method of producing a fabric having a halogen free plasma coating, especially free of per- and polyfluorinated substances (PFAS), having hydro- and oleophobic characteristics as well as to provide a fabric comprising a halogen free plasma coating, especially free of per- and polyfluorinated substances (PFAS), formed thereon having a hydro- and oleophobic characteristics.

According to the invention this object is achieved on the one hand by a method having the features of claim 1 and by a fabric, like a fabric, having the features of claim 14.

Preferred embodiments of the invention are stated in the respective dependent claims.

According to the inventive method in a step DHF a plasma coating is deposited on the fabric by means of plasma polymerization of a halogen free precursor monomer by plasma-enhanced chemical vapor deposition method (PECVD). The halogen free precursor monomer are organosilane, siloxane and/or hydrocarbon precursors. Further the plasma-enhanced chemical vapor deposition is carried out as a low-pressure plasma processes under protective atmosphere. The fabric comprises of a woven monofilament fabric of polymeric material having a filament diameter between 10 µm to 150 µm and a mesh opening between 5 µm and 200 µm.

A basic idea of the invention is the identification of the influence of fabric geometry on the achievement of a good oleophobic textile surface. Textile or fabric structure and construction in turn depend on many factors, such as the type of weave, the type of fiber content, the fiber fineness, the mesh size, i.e., the number of threads per centimeter. Compared to flat surfaces (films, polymeric solids, etc.), fabrics have complex architectures that actually consist of two surfaces, one of which is macroscopic and visible to the naked eye. It has been found that the degree of oleophobicity is closely related to the textile structure and weave construction. Thus, in addition to plasma process parameters and coating properties, the capillary phenomenon is strongly influenced by the mesh geometry, in particular the mesh opening (space between two adjacent filaments) and the yarn (filament) diameter. The fabric may comprise regular openings of a square or rectangular configuration. Surprisingly, according to the invention, it was discovered that a fabric consisting of a woven monofilament fabric of polymeric material having a filament diameter between 10 µm and 150 µm and a mesh opening between 5 µm and 200 µm, with only a plasma deposition coating based on organosilane, siloxane and/or hydrocarbon precursors, which are considered to provide only hydrophobic properties, also provides oleophobic properties.

Plasma processed organosilicons as in step DHF result in a complex plasma-phase chemistry and in the tunability of films composition. In fact, depending on the number of organic moieties (CHx), a silicone-like character can be obtained when a great number of (CHx) is present in the film which exhibits hydrophobic properties or a SiO₂-like inorganic coatings in case an oxidant (such as oxygen) is added to the feeding gas which results hydrophilic surface.

Liquid precursors which can been used to deposit Si-based coatings are Hexamethyldisiloxane (HMDSO), Tetramethyldisiloxane (TMDSO), Divinyltetramethyldisiloxane (DVTDMSO), Tetramethylsilane (TMS). Such a monomer has high vapor pressure, non-toxic and can be processed at low temperatures. It can be used for the deposition of hydrophobic coatings onto fabric and fibers due to the retention of -CH₃ groups within a Si-O network. Carbon-rich, plasma polymerized TMDSO (pp-TMDSO) from pure TMDSO using PECVD process, shows promising mechanical properties such as low internal stress, good adhesion to substrate.

On the other hand, an organic character of plasma polymerized TMDSO can be obtained using hollow cathode plasma polymerization from gaseous mixtures of TMDSO/N₂/Ar (He). Explaining the hollow cathode system, plasma is generated in a narrow inter-electrode gap, normally with coaxial geometry, and it is blown outside that region by gas flow directly onto the substrate: the plasma treatment or deposition thus occurs in downstream mode as described in WO 2022/171581A.

Developed both types of coatings of the DHF step show an excellent hydrophobic and a good oleophobic barrier performance on monofilament fabric, whereas mesh opening, and mesh diameter plays an important role in obtaining oleophobic properties.

In one embodiment the method can comprise the additional step DLC of coating the fabric by means of sputtering of a carbon target by PVD process using an Argon plasma and/or using a hydrocarbon gas by PECVD method, wherein the step DLC is carried out before step DHF. In the DLC step, an amorphous hydrogenated diamond-like carbon film is deposited on the fabric.

Amorphous hydrogenated diamond-like carbon films (also known as a-C:H or DLC) have shown several properties like high hardness, high wear resistance, chemically inert, oxidation resistant, thermal stability, highly crosslinked and low friction coefficient. Besides the coating properties it is an important aspect that DLC coatings can be deposited at low substrate temperatures, e. g. on temperature sensitive polymeric materials like monofilament fabrics. DLC coatings, consisting of a highly crosslinked network of carbon and hydrogen atoms, commonly have high compressive stress. Such high stress values can cause poor adhesion with the substrate and limit the practical application. As DLC coating has high adhesion to substrate, the coating can be used as adhesion promoting layer for the DHF coating.

According to the invention two different methods to deposit DLC coating are proposed. One of the methods is to deposit DLC films is the radio-frequency RF glow discharge of hydrocarbon gases with negatively self-biased voltage applied to substrate by means of PECVD method. The second type of DLC coating can also be deposited by means of PVD sputtering. The used target consists of carbon or pure graphite and the working gas is an argon during sputtering process.

The DHF step can be performed subsequent to the DLC step in the same reactor. This is particularly advantageous when the PECVD method is used to produce the amorphous hydrogenated diamond-like carbon film. Alternatively, the DHF step can be carried out in a separate reactor with a time delay between these steps.

The method may also comprise an APL step of creating an adhesion promoting layer by plasma polymerization of hydrocarbon gas and/or a mixture of hydrocarbon, reactive and inert gases, wherein the APL step is carried out before step DLC or before step DHF if no DLC step is carried out.

In contrast to conventional polymerization, plasma polymerization can be performed using any kind of hydrocarbon gases. Mainly methane (CH₄), ethylene (C₂H₄) or acetylene (C₂H₂) are used. The amorphousness of the obtained adhesion promoting layer is related to the degree of cross-linking. Independently of the used monomer, saturated and unsaturated monomers show different deposition rates. As an example, it was found that plasma polymers derived by acetylene appears to be yellowish due to unsaturated bond left within the film structure. Methane has resulted in reduced deposition rate. In order to obtain nitrogenated hydrocarbon adhesion layer, reactive gas like ammonia has been mixed with ethane during plasma process. Some more examples of reactive gases are N₂, CO₂, CO, N₂O.

The advantage of the adhesion promoting treatments with respect to the subsequent layers deposited on the fabric, such as in the DHF or DLC step, is that these layers and coatings have a better adhesion and are therefore more durable during the use of the fabric.

Hydrocarbon layer acts as adhesive joints increasing the DHF or DLC cohesion. Moreover, admixture of reactive gas to hydrocarbon plasma results incorporation of functional groups increasing the surface energy of the fabric. As a consequence, this functionalized surface layer acts as chemical anchoring (bonding) with the subsequent coating like DHF.

As an alternative to the APL step the method may also comprise carrying out an APT step of creating an adhesion promoting surface by using plasma etching, an ion beam irradiation and/or UV imprinting, wherein the APT step is carried out before step DLC or before step DHF if no DLC step is carried out.

Plasma etching processes can be used to produce patterns from the nanometer to the micrometer range. The very severe requirements in terms of etch rate, selectivity, profile control and surface damage plasma-etching processes lead to, have been at the origin of the development of many studies by means of plasma diagnostics and surface analysis, as well as the development of new etching devices. The key parameters of the plasma-surface interaction vary with each material upon the gas mixture and the ion bombardment. Mostly, the dominant parameter is the ratio of the neutral flux to ion energy flux.

In the plasma etching (also known as reactive ion etching) of organic polymers like fabric, a gas mixture of oxygen and tetrafluoromethane (CF₄), for an example, is used to create oxyfluoride ions (OF-), and this ion is a highly reactive etching agent for polymeric substances like monofilament, particularly for cutting the carbon-carbon bonds in a polymer backbone. It has been found that the applied pressure and bias voltage determine the etching effect on the surface morphology of bulk polymer.

Another ion milling technique is ion beam irradiation, commonly referred to as ion beam milling or ion beam sputtering. It removes material off the target surface by using a focused, precisely defined beam of ions, often made of inert gas ions like argon. Ion beam irradiation, which differs from reactive ion etching in that it doesn't rely on chemically reactive gases, is a wholly physical sputtering process. These ion sources produce a high-density, collimated beam of ions with well-controlled energy and direction. The ion beam is directed towards the target surface, where the energetic ions collide with the surface atoms, causing them to be sputtered away. Ion beam etching offers several advantages, such as high etching rates, excellent depth control, and the ability to create anisotropic etch profiles. Since the process does not involve chemically reactive species, the etching process is relatively clean and less prone to contamination.

UV imprinting is also a common method to fabricate nano-microstructured surface. Additionally, or alternatively prior to the deposition in steps DHF and/or DLC a two-step pre-treatment of polymeric fabric can be performed, wherein in a first step the polymeric fabric is coated with UV curable imprint resin using gravure and/or slot die coating method and in a second step a surface patterning is performed using UV imprinting and/or hot embossing method.

The etched or irradiated or imprinted surface results in obtaining a good adhesion to the subsequent coating via mechanical interlocking as well as a better roll-off effect of water and oils when modified surface is then coated with the step DHF.

Preferably in step DHF the halogen free hydro- and oleophobic coating is deposited on the fabric having a thickness from 30 nm to 300 nm, preferably between 50 nm to 150 nm. It has been shown that a coating in this thickness range is already sufficient to provide good oleophobic and hydrophobic properties.

Advantageously in step DLC the hydrophobic carbon coating is deposited on the fabric having a thickness from 5 nm to 200 nm, preferably between 10 nm to 80 nm. Even such a thin coating significantly improves the adhesion and chemical resistant properties of the fabric.

Beneficially in step APL an adhesion promoting coating is deposited on the fabric having a thickness from 5 nm to 100 nm, preferably 10 nm to 60 nm. The adhesion properties of the subsequent coating can already be significantly improved with such a thin layer.

The advantage of these thin layers is that the open mesh size of the fabric is not significantly affected by these coatings in the steps described above. As a result, the air permeability is hardly impaired.

The plasma treatment can be performed in a plasma chamber, in case of a fabric having a plurality of rollers and/or expanders in a roll-to-roll system. Depending on the electrode arrangement, single side or both sides treatment of the fabric can be performed. For the PECVD especially in steps DHF, DLC and/or APL different types of electrodes such as hollow cathode, parallel plates, drum can be used as plasma source which are connected to one or more of alternative current (AC), direct current (DC), pulsed DC, continuous radio-frequency (RF), pulsed RF etc.

The degree of crosslinking of the plasma polymer film strongly depends on the energy input. Power density also depends on the plasma machine configurations such as electrode arrangements. For plate or drum-like electrode arrangement, a plasma power during step DHF and step APL can be lower than 1 W/cm² of electrode surface, preferably lower than 500 mW/cm² of electrode surface or more preferably lower than 200 mW/cm² of electrode surface. For hollow cathode electrode setting, the power density of plasma can vary between 2.5 kW to 18 kW per linear meter of plasma. Besides the used electrode arrangement, the concept of the gas supply into the reactor chamber is also relevant for the preferred plasma power.

A plasma power during step DLC for PECVD process may be lower than 1 W/cm² of electrode surface, preferably lower than 500 mW/cm² of electrode surface or more preferably lower than 200 mW/cm² of electrode surface and for PVD sputtering is between 2000 to 8000 mW/cm².

In a preferred embodiment, the fabric has a filament diameter between 10 µm to 100 µm, particularly preferably 19 µm to 50 µm. Alternatively or additionally, the fabric has a mesh opening size (space between two adjacent filaments) between 5 µm and 150 µm, particularly preferably 19 µm to 125 µm.

It has been shown that the filament diameter in particular must not fall below a lower limit, as otherwise the capillary effects increase and the oleophobic properties deteriorate significantly. Similar effects also result from different mesh sizes. Here, too, there is a preferred area where the capillary effects are at their lowest.

In a preferred embodiment the fabric being plasma coated in step DHF has a hydrophobic character corresponding to water contact angle between 110° to 160° and oleophobic character corresponding to Diiodomethane contact angle between 80° to 140° and Hexadecane contact angle between 40° to 120° according to DIN 55660-2:2011-12 and oil grade up to 4 according to DIN EN ISO 14419:2010 (or AATCC 118).

In a further preferred embodiment, the fabric being coated in step DLC is chemically inert and resistant to acid, alkali and organic solvents and has a hydrophobic character corresponding to water contact angle between 90° to 140° according to DIN 55660-2:2011-12.

In addition to the steps explained before the method may further comprise a step PT of a pre-treatment of the fabric by means of an atmospheric or low-pressure plasma using non-polymer forming an inert gas and/or a reactive gas wherein step PT is carried out before step DLC or before step DHF if no DLC step is carried out. If a step APL is used preferably the step PT is also carried out before them. Step APT may be carried out without conducting a PT step before.

To clean the fabric and improve adhesion with the plasma coating, a pre-treatment of the polymeric fabric can be performed prior to the deposition of the functional layer as in step DHF or DLC by means of atmospheric and low pressure plasma technologies using nonpolymerizable gases like argon, helium, nitrogen, oxygen, and tetrafluoromethane gases and/or a gaseous mixture thereof. Compared to atmospheric pressure plasma, the low pressure plasma pre-treatment has several advantages. Gas at low pressure allows the acceleration of free electrons when driven by an external source (e.g. RF generator). As a consequence, highly reactive and activated molecular species such as chemical radicals, ions, electrons etc. can be created by ionization, fragmentation (dissociation), excitation, UV radiation, etching reactions etc. These species chemically and physically react with the polymer surfaces thus altering the surface properties and surface morphology in the topmost layers. Surface activation changes from surface cleaning, radical formation and atom implantation to surface etching; it depends on different process parameters such as energy input, for example. An oxygen containing gaseous mixture (Ar/He with O₂) was found to be more efficient when compared to pure inert gases (Ar, He etc.) in order to remove organic contaminants by oxidizing polymer surfaces, and by producing plasma degradation products such as hydrogen, steam, carbon dioxide etc.

In principle, the previously explained steps PT, APL or APT, DLC and DHF can be carried out in any arbitrary sequence as well as it is possible to carry out several steps more than once. In one preferred embodiment, the steps are carried out in the following order: step PT, step APL or APT, optionally step DLC, and step DHF. It has been recognized that treating an object to be provided with an oleo- and hydrophobic coating by the steps in the previously defined sequence, leads to an extraordinary result.

Based on the inventive method, it is possible to produce a fabric comprising a halogen free plasma coating, especially free of per- and polyfluorinated substances (PFAS), according to standard IEC 62321-3-2:2020, EN 14582:2016 and/or ASTM D7359:2018 formed thereon having a hydro- and oleophobic wherein the fabric comprises of a woven monofilament fabric of polymeric material having a filament diameter between 10 µm to 100 µm and a mesh opening between 5 µm and 200 µm.

This fabric can for an example be used as protective vent in mobile devices, as filters for many applications: acoustic vents, ventilation filters, fuel filtration, water separation, clothing, packaging, building and electronic seals/circuit boards, shoes, wound dressings or facial masks. The invention is further directed to electronic or electrical appliances such as mobile phones, portable media players, high fidelity equipment, tablets, laptops, portable devices of any kind and televisions. The fabric according to the invention can be used for diverse venting applications in healthcare such as infusion / transfusion / blood filters, matrasses, pillows, duvets, beddings, ventilation filter for (electric) equipment (in and/or out), (surgical) masks, (surgical) coats, intravenous in-line filter sets, pressure filtration equipment, in particular in in medical devices, room ventilation and venting barrier media for industrial applications.

The fabric according to the inventions is preferably a woven monofilament fabric in which the filaments are made of the same material. Alternatively, fabrics with filaments of different materials could be used.

The fabric could be made of and/or comprise one or more of the following materials or combinations thereof: polyvinyliden chloride (PVDC), polyhexamethylen adipamide (PA6.6), polydodecanamide (PA12), polypropylene (PP), polycaproamide (PA6), polyethylene terephthalate (PET), ethylene monochlor trifluor ethylene (E-CTFE), ethylene tetrafluor ethylene (ETFE), polyeth-ylene (PE), polyoxymethylen (POM), polycaprolactone (PCL), polysulfone (PS), chitosan (CH), polyvinylbutyral (PVB), 1-dodecyltrimethylammonium bromide (DTAB), chlorhexidine (CHX), benzyltrime thylammonium bromide (BTAB), polyacrylate, polyethylene (PE), high density PE, fluorized ethylenepropylene (FEP), bi-component (PA6/PA12), polybutylene terephthalat (PBT), polyetheretherketone (PEEK), polyacrylonitrile (acrylic fibers) (PAN), bicomponent, PET flame retardant (PET/PBT), polyunde-canamide (PA 11), polyphenylensulfide (PPS), poly-hexamethylen sebacinamid (PA6.10), aramide (AR), polyethylene naphtalate (PEN), polyamide carbonfiber (PA/CF), polyester carbonfiber (PET/CF), polyester staple fiber / metalfiber (PET/MT), carbon fiber (CF), copper (CU), polyimide (P84), copper / silver (CU/AG), polycarbonat (PC), aliphatic polyamide, aromatic polyamide, polyurethane (PU), polyvinyl alcohol (PVA), polylactide (PLA), polybenzimidazole (PBI), polyethylenoxide (PEO), poly(butylene terephthalate), polyvinylchloride (PVC), cellulose, cellulose acetate (CA), polypropylene (PP), PVA/silica, PAN/TiO₂, PETFE polyetherimide (PEI), polyaniline, poly(ethylene naphthalate), styrenebutadiene rubber, polystyrene, poly(vinyl butylene), polymethylmethacrylate (PMMA).

### Plasma technology

Interest in plasma processes has been increasingly growing, especially low temperature plasma, for the modification of the surface properties of polymeric materials without changing the nature of the bulk of the substrate. Plasma techniques generates radicals and oxidizes the surface (i.e., hydro/oleophobic surface); it changes the topography: adhesion, repellence properties, roughening of the surface; it permits the cleaning of surfaces which leads to an increase of quality printing, dye-uptake, painting etc. In the field of textile finishing, plasma technology shows distinct advantages because it is a dry and environmentally friendly process. In addition, due to enormous variety of feeding gases which can be used to perform a great number of surface chemistry, a multiple variety of chemically functions can be incorporated onto the textile surface to obtain different chemical and physical features. Non-thermal plasma has been intensively used for the practical industrial requirements to provide high quality, high productivity, low cost and environmentally clean surface treatment processes.

Furthermore, plasma polymerization or plasma deposition processes provide a versatile route to design materials with tunable functionalities. Due to its exclusive properties of plasma polymer smart coatings like tunable wettability, self-cleaning and antireflective make them prominent in diverse applications such as biomaterials, drug delivery, adhesion, protective coatings, microelectronic devices, oil-water separation and thin film technology.

### PECVD Process

Plasma enhanced chemical vapor deposition (PECVD) is the suitable polymerization method that utilizes precursors in either liquid or gas form. By means of such a quite controlled polymerization process pinhole free, cross-linked and dry deposition of the polymer is ensured and the difficulties faced during wet chemical polymerization such as non-uniform coatings, the impurities by the solvent produces defective coatings due to the presence of solvent can be avoided.

Plasma treatment improves the crosslinking degree in polymer when compared to classical polymerization. Explaining more the plasma polymerization process; monomer precursors which are already vaporized are pumped to the vacuumed plasma chamber. The energy input generates then excited electrons during glow discharge and lead to break molecules into free electrons, ions, radicals and excited molecules. Afterwards these free radicals and excited molecules recombine, condense and polymerize on the substrate, the ions and electrons crosslink or form chemical bonds with the already deposited polymer, so the properties of plasma polymers are not only determined by precursors but also by the deposition parameters.

The plasma treatment of textile or other materials can be applied as textile finishing process i.e. for technical and medical textiles as well as for composite materials to improve their surface properties like water and oil repellency. This is also possible for other materials and compact objects. Compared to conventional wet-chemical textile finishing, plasma technology shows advantages regarding environmental issues. With the PECVD treatment, e.g. improvement of adhesion characteristics, increasing hydrophilicity / hydrophobicity, introducing special functional groups on the surface, or modifying the surface morphology can be obtained.

In plasma deposition, which is commonly known as plasma polymerization or PECVD, a very thin polymer layer (nano-scaled) can be deposited on the substrate surface. The layer is formed through polymerization of an organic precursor gas, which is directly polymerized on the substrate surface. In contrast to classic polymerization, plasma polymerization can use every monomer gas or vapor which is not limited to their reactivity. The plasma polymer shows unconventional polymerization behavior with branched and randomly terminated chains and a high degree of crosslinking.

The bulk structure of plasma polymers is completely irregular, far from that of conventional polymers. Plasma polymer coating (nanothin film) differs from conventional polymer by a high density of functional groups per volume, a highly cross-linked and branched plasma polymer network, a nanometer thick coating, a high adhesion of coating to the substrate and a no change of bulk properties of the substrate, which can be a polymeric fabric.

There are different types of power sources can be used for DHF, APL, APT, DLC processes. RF plasma sources are by far the most common. Most RF sources use the 13.56 MHz industrial standard frequency. Among these, there are three main types: capacitively coupled plasmas or CCPs, also called reactive ion etchers (RIEs); inductively coupled plasmas (ICPs), also called transformer coupled plasmas (TCPs); and helicon wave sources, which are new and can be called HWS. A plasma RF generator produces a high-power RF signal and is one of the key front-end sub-systems of a larger industrial plasma processing system. The number of applications for industrial plasma processing has exploded over the past 20 years or so, and multiple industries now benefit from using plasma processing.

Pulsed RF power for plasma processing has been introduced as solution the avoid the problems which have been faced with CW RF power like charge-up damage and reflected power from the chamber. Although the delivery process for pulsed RF power is more complex than the delivery process for CW RF power. WO 2001/084591 A2 provides method for overcoming the said problems relating to the delivery of pulsed RF power to a plasma processing chamber.

The DC and low frequency discharges can work in continuous and pulsed mode. Pulsed DC power are generated in highly asymmetric electrodes configuration, like sharply pointed needle or thin wire opposing flat planes or large diameter cylinder (drum), when a high voltage is applied to the smaller electrode an intense electric field is generated around it, so plasma ignites in the form of streamers.

Although AC plasma generators were thought to be more efficient and less expensive, prior art AC systems were found to be inherently unstable. One source of this instability is the fact that if the arc is pulsed in a single phase system, the arc goes out during each half cycle. Therefore, the arc must be initiated 120 times per second.

### Test methods

In order to determine the halogens concentrations, in particular PFAS concentrations, the following test methods were used. All these three standards cover to detect Total Fluorine (TOF). Extractable Organic Fluorine (EOF) would be the same CIC analysis, but a methanol extract would be combusted instead of the sample directly. TOF and EOF results cannot be compared directly, normally the TOF results are much higher (circa by factor 2 to 12).
- IEC 62321-3-2:2020: Specifies the screening analysis of fluorine, chlorine and bromine in polymers and electronics using combustion ion chromatography (CIC).
- EN 14582:2016: This standard specifies a combustion method for the determination of halogen and sulfur contents in materials by combustion in a closed system containing oxygen (calorimetric bomb), and the subsequent analysis of the combustion product using different analytical techniques.
- ASTM D7359:2018: Standard Test Method for Total Fluorine, Chlorine and Sulfur in Aromatic Hydrocarbons and Their Mixtures by Oxidative Pyrohydrolytic Combustion followed by Ion Chromatography Detection (CIC). This method might be comparable to OEKO-TEX STANDARD 100 method.

In the following the invention is described further by way of a preferred exemplary embodiment illustrated schematically in the accompanying drawings, wherein show:
- Figure 1: a combination of a schematic flow chart of the inventive method including an illustration of the different deposited films on a fabric;
- Figure 2: a combination of a schematic flow chart of the inventive method including an illustration of the different results on a fabric; and
- Figure 3: two AFM images of plasma etched PEEK fabric

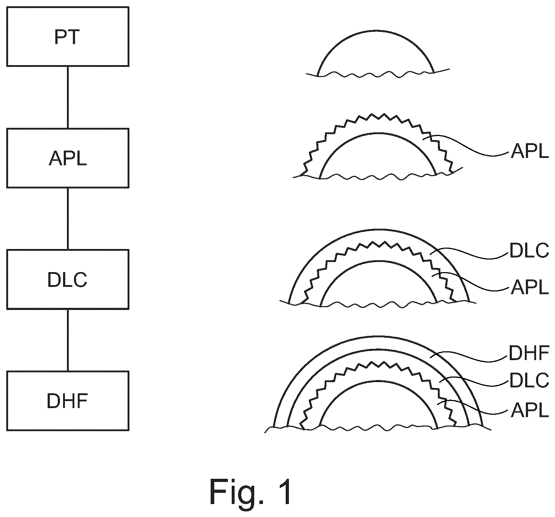

On the left side of figure 1 a schematic flow diagram is shown, comprising several steps according to the invention. The right side of figure 1 illustrates the deposited layers on a fabric, wherein only one monofilament is show, for clarity reasons.

According to this embodiment, a step PT is performed first. In this step, a plasma pre-treatment is carried out on the surfaces of the fabric, which should be a woven monofilament fabric of polymeric material on which the various subsequent layers are to be deposited. The plasma treatment is preferably carried out in a closed chamber with a low pressure atmosphere. The aim of the treatment is to clean the surface of the fabric so that the subsequent polymers can be deposited more easily. Depending on the power used, the plasma treatment can also roughen the surface of the substrate to make it easier for subsequent layers to adhere. Sometimes this roughening is seen as the creation of micro-grooves in the substrate material.

The PT step can be followed by either an APL or DHF step to improve the adhesion of subsequent layers. In the APL step a layer can be deposited on the fabric by plasma polymerization of hydrocarbon gas and/or a mixture of hydrocarbon, reactive and inert gases.

As can be seen on the right side, after the cleaning of step PT, an APL layer is applied onto the fabric.

Following to the APL step, a step DLC is optionally executed wherein amorphous hydrogenated diamond-like carbon coating is deposited on onto the previous APL layer. Due to the combination of these two layers, shown on the right side only in a very simplified manner, it can be ensured that the whole surface of the substrate is coated. Normally the thickness of the layers may not be this constant.

Following the DLC step, a step DHF is executed, a non-fluorinated polymer coating is performed which can be based e. g. on organosilanes (trimethyl silane etc.), siloxans (hexamethyldisiloxane, divinyltetramethyldisiloxane, tetramethylsilane etc.), hydrocarbon (methane, ethane, acetylene etc.) and a mixture of thereof.

Preferably, the object to be coated is kept in the chamber with the low pressure or with the vacuum during the entire treatment process.

After the step DHF is executed, in principle the coated object can be used or delivered to further processing.

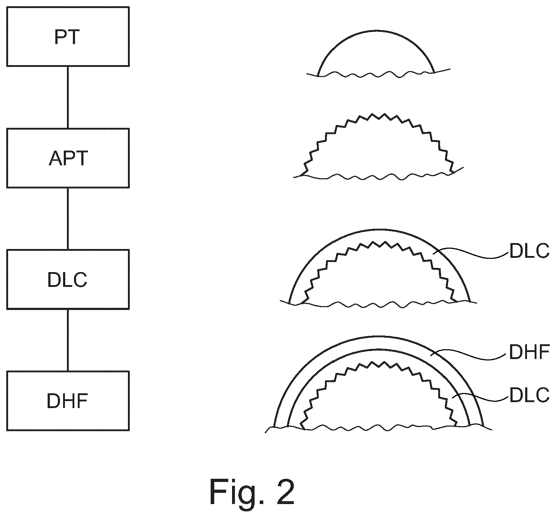

Fig. 2 shows an alternative treatment of a fabric. The main difference regarding the method steps of Fig. 1 is that instead of an APL step an APT step is carried out.

In the APT step, the surface of the monofilament is roughened, which also improves the adhesion of subsequent layers. This can be achieved by plasma etching, ion beam irradiation and/or UV imprinting. The PT step is not necessary prior to APT step.

The other steps of this example correspond to those of Fig. 1. Hence step APL and step APT can be seen as alternatives.

In view of the oleophobic properties the object to be coated, should be a woven monofilament of polymeric material having a filament diameter between 10 µm to 100 µm and a mesh opening between 5 µm and 200 µm.

### Used fabrics

Eight different fabric articles have been analyzed in order to find out the influence of fabric construction in determining hydro- and oleophobic properties. Table 1 summarizes the fabric parameters as well as the features. This information provides the basis for understanding their behavior and developing new materials with specific surface properties. For example, a tightly woven mesh which has very low mesh opening of 5 µm and filament diameter of 34 µm can be seen in fabric A. On the contrary, a very opened mesh with a mesh opening of 200 µm and filament diameter of 100 µm can be seen in fabric H. In addition, also a composite article (I) was considered, having mean pore size of 0.70 µm and air permeability of 35 l/m²s@200Pa which is made from a mesh article D, a membrane layer and a knitted material. A low surface tension liquid (e.g. oil) possess increased capillary action for such a composite material.

**Table 1. Comparative example fabric properties**

| Fabric | Material | Filament dia- meter [µm] | | Mesh opening [µm] | | Open area [%] | Air permeability [l/m².s@ 200Pa] | Fabric thickness [µm] | Weave pattern |
|---|---|---|---|---|---|---|---|---|---|
| | | Warp | Weft | Warp | Weft | | | | |
| A | PET | 34 | 34 | 5 | 5 | 1.5 | 80 | 85 | TW |
| B | PET | 24 | 19 | 19 | 19 | 22 | 2000 | 42 | TW |
| C | PET | 27 | 27 | 25 | 25 | 22 | 2300 | 52 | TW |
| D | PA66 | 40 | 40 | 50 | 50 | 31 | 4300 | 60 | PW |
| E | PET | 32 | 32 | 64 | 64 | 44 | 7600 | 45 | PW |
| F | PET | 86 | 86 | 150 | 150 | 40 | 5400 | 135 | PW |
| G | PEEK | 38 | 38 | 195 | 195 | 70 | 7500 | 58 | PW |
| H | PET | 100 | 100 | 200 | 200 | 43 | 8700 | 170 | PW |

| | | | | | | | | | |
|---|---|---|---|---|---|---|---|---|---|
| wherein TW is twill weave and PW is plain weave | | | | | | | | | |

### Example 1

A contact angle of three liquids and an oil drop test have been measured respectively according to DIN 55660-2:2011-12 and DIN EN ISO 14419:2010 on eight different articles which have been coated with a siloxane based coating (DHF). Hydrophobicity is evaluated by measuring water contact angle, whereas the oleophobicity is determined by measuring contact angle with Diiodomethane and Hexadecane and by oil drop test. As can be seen in table 2, it was found that tightly woven fabric yielded higher capillary and as a consequence, a very low to moderate oil repellency can be obtained (e.g. fabric A). In contrast, a good oleophobic surface can be obtained by adjusting the mesh opening and yarn diameter due to the inhomogeneous wicking with low surface tension liquid like oils (e.g. fabric C-F). A moderate oleophobic modification can be obtained on very opened mesh because of droplet penetration (e.g. fabric G-H). These findings suggested that both the mesh geometry is important parameters, in addition to plasma process, which must be considered, when performing plasma polymerization of siloxane coatings, as the resulting oleophobicity can vary quite significantly.

**Table 2. Contact angle and oil grade results of different fabric geometries obtained after DHF step**

| Fabric | Contact angle [°] according 2:2011-12 to DIN 55660- | | | Oil drop test according to DIN EN ISO 14419:2010 [grade 0-8] |
|---|---|---|---|---|
| | Water [°] | Diiodome-thane [°] | Hexadecane [°] | |
| A | 122 | 104 | 9 | 0 |
| B | 132 | 113 | 66 | 2 |
| C | 128 | 117 | 70 | 3 |
| D | 127 | 126 | 75 | 3 |
| E | 130 | 119 | 79 | 3 |
| F | 124 | 106 | 75 | 3 |
| G | 138 | 119 | 42 | 2 |
| H | 131 | 112 | 41 | 2 |

### Example 2

It is important to protect coatings from destructive and harsh conditions during application. Abrasive force is an example can ablate deposited coating. Therefore, abrasion resistance is one of the limiting factors in determining the lifetime of a product. In order to make sure that the plasma coating (PT followed by DHF steps) adheres well with a substrate surface which is a multi-layer composite membrane material (I), an abrasion test has been performed according to EN ISO 12947 Serie und EN ISO 12945-2. Table 3 reveals a significant abrasion resistance of the coating. There is almost no decrease in contact angles with two liquids which was measured according to DIN 55660-2:2011-12, demonstrating high coating adhesion to the object. However, we found rather poor oleophobic properties on the coated composite membrane due to higher capillary effect. A composite membrane according to the invention may comprise a woven base layer and a superimposed nano-fiber layer like an e-spun membrane. The e-spun membrane produced consists of numerous nanofibers interweaved and overlapped with each other to form fibrous and porous structures inside which results very high capillary action with low surface tension liquids like oils.

**Table 3. Comparative example contact angles with two liquids before and after abrasion**

| Fabric | Treatment type | Contact angle DIN 55660-[°] according to 2:2011-12 | | Oil drop test according to DIN EN ISO 14419:2010 [grade 0-8] |
|---|---|---|---|---|
| | | Water [°] | Diiodomethane [°] | |
| I | PT+DHF before abrasion | 129 ±2 | 110 ±2 | 0 |
| | PT+DHF after abra- sion | 128 ±2 | 109±2 | 0 |

### Example 3

In order to evaluate water separation efficiency, the test has been performed according to ISO/TS 16332 standard on PT+DHF and DHF coated polyester article. It can be seen in the table 4 that an excellent water separation efficiency is obtained with a pre-treatment step (PT) followed by the DHF step compared to DHF without PT step.

**Table 4. The developed coating shows excellent water separation efficiency**

| Fabric | Treatment type | Water separation top side [%] | Water separation bottom side [%] |
|---|---|---|---|
| B | PT+DHF | 93.3 | 91.6 |
| C | DHF | 84.5 | 85.2 |

### Example 4:

In order to make sure that the DLC coating can be used as adhesion promoting layer for the DHF coating and the resulted coating (DLC+DHF) adheres well with the substrate, an internal washing test has been performed at 40°C temperature for 47 min. The washing cycles are respectively 0XW: no wash, 1XW: one wash cycle and 10XW: ten wash cycles. The contact angle with three liquids and an oil drop test have been measured respectively according to DIN 55660-2:2011-12 and DIN EN ISO 14419:2010. Table 5 reveals a significant washing resistance of the coating. There is a little decrease in contact angles with two liquids demonstrating high coating adhesion to the object. It can also be seen that including the DLC coating as an undercoat for the DHF step resulted in higher contact angle, meaning an improved wash resistance can be obtained with DLC step followed by DHF step. The oil repellency of the washed object has also been evaluated 8 different liquid oils and the results show a little decrease in oil repellency even after 10 washing cycles. Therefore, based on the invention it is evident from this data that a robust and reliable coating on polymeric fabric with excellent hydro- and oleophobic characteristics could be obtained.

**Table 5. Comparative example contact angles with two liquids and oil drop test before and after washing**

| Fabric | Treatment type | Washing cycles (40°C/47min) | Contact angle [°] according to DIN 55660-2:2011-12 | | Oil drop test according to DIN EN ISO 14419:2010 [grade 0-8] |
|---|---|---|---|---|---|
| | | | Water contact angle [°] | Diiodomethane contact angle [°] | |
| C | DHF | 0XW | 128 ±1 | 117 ±3 | 3 |
| | DHF | 1XW | 122 ±2 | 110 ±2 | 2 |
| | DHF | 10XW | 123±3 | 110 ±2 | 2 |
| | DLC+DHF | 0XW | 137 ±1 | 124 ±1 | 3 |
| | DLC+DHF | 1XW | 130 ±1 | 124 ±1 | 3 |
| | DLC+DHF | 10XW | 126 ±2 | 123 ±1 | 2 |

### Example 5

In addition to performance and functionality tests, the coating conformity for medical applications such as endotoxin and hemocompatibility testing on coated fabric according to the invention were performed.

Endotoxin testing is performed to determine the accessibility of the product for medical applications. The endotoxin limit is calculated according to Pharmacopoeia (EP 10, January 2020 and USP 42, May 01, 2019 <85>. Both DHF and PT+DHF coated articles contain less than the endotoxin limit concentration and have passed the test, as can be seen in table 6.

Hemocompatibility of blood-contacting materials is also one of the most important criteria for medical applications. The interactions between newly developed coated materials and blood has been extensively analysed, in accordance with ISO 10993-4 and ISO 10993-12, to prevent activation and destruction of blood components in applications. The hemocompatibility analysis of the coated articles are summarized in the below table 6 and all coated articles passed the test.

**Table 6. Endotoxin and hemocompatibility test**

| Fabric | Treatment type | Test type | Tested parameter | Results | Specification (limit value) | Pass / Fail |
|---|---|---|---|---|---|---|
| C | DHF | LAL | Endotoxin [EU/ml] | 0,063 | ≤ 0.125 | PASS |
| | | | PPC (positive product control) [%] | 121 | 50-200 | PASS |
| | | Hemolysis | OD (optical density) | 0,003 | ≤ 0.03 | PASS |
| I | PT+DHF | LAL | Endotoxin [EU/ml] | 0,043 | ≤ 0.125 | PASS |
| | | | PPC (positive product control) [%] | 117 | 50-200 | PASS |
| | | Hemolysis | OD (optical density) | 0,002 | ≤ 0.03 | PASS |
| E | DHF | LAL | Endotoxin [EU/ml] | 0,060 | ≤ 0.125 | PASS |
| | | | PPC (positive product control) [%] | 111 | 50-200 | PASS |
| | | Hemolysis | OD (optical density) | 0,004 | ≤ 0.03 | PASS |

### Example 6

Plasma etching is done with a gaseous mixture of CF₄ and oxygen using low temperature and low-pressure plasma system. A morphological characterization of the etched surface was done by AFM. Rq is the root-mean-squared roughness and Ra is the roughness average. The average roughness is the area between the roughness profile and its mean line. It is evident that direct bias plasma has proven to be very efficient to roughen the textile fabric. The roughness of the small mapping seems to be more consistent. The roughness of the large mapping could be influenced by the large surface features, such as the grooves in the maps.

**Table 7. Comparative example of etched samples investigated by Atomic Force Microscopy (AFM)**

| Fabric | Etching type | Plasma power | Tested area | | | |
|---|---|---|---|---|---|---|
| | | | 2µm x 2µm | | 10µm x 10µm | |
| | | | Rq (nm) | Ra (nm) | Rq (nm) | Ra (nm) |
| G | Direct Bias plasma | 500V | 126 | 101 | 85 | 68 |
| | Remote Plasma | 1300W | 29.6 | 22.7 | 49.6 | 38.3 |
| | Untreated | None | 4.0 | 3.1 | 10 | 7 |

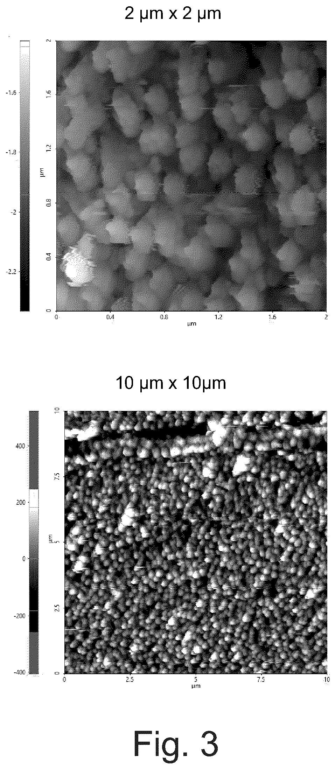

Topographical changes made by etching process are further investigated by AFM images, which are shown in Fig. 3. Fig. 3 shows AFM images of plasma etched PEEK fabric (direct bias, 500 V). The homogeneous texturing and roughening of the PEEK surface due to the etching conditions can be seen.

### Example 7:

The Total Fluorine screening for Perfluoroalkyl and Polyfluoroalkyl Substances (PFAS) on coated sample was performed with reference to IEC 62321-3-2:2020. The result may include inorganic and/ organic fluorine content. There is a possibility that the total fluorine content does not come from PFAS. It is evident that all treatment steps (PT, APT, APL, PT, DLC, DHF) and their combinations are free of PFAS.

Table 8 shows no PFAS (eg. Fluorine) has been detected on the hydro- and oleophobic coated fabric

**Table 8. Detection of PFAS according to IEC 62321-3-2:2020**

| Fabric | Treatment type | Limit | Unit (s) | MDL | Test result PFAS expressed as Fluorine |
|---|---|---|---|---|---|
| A | DHF | 50 | mg/kg | 20 | ND |
| C | DLC+DHF | 50 | mg/kg | 20 | ND |
| E | PT+DHF | 50 | mg/kg | 20 | ND |
| I | APL+DHF | 50 | mg/kg | 20 | ND |
| I | APT+DHL | 50 | mg/kg | 20 | ND |

| | | | | | |
|---|---|---|---|---|---|
| wherein 1 mg/kg = 1 ppm = 0.0001% MDL = Method Detection Limit ND = Not detected (<MDL) | | | | | |

### Example 8

A contact angle of two liquids and an oil drop test have been measured respectively according to DIN 55660-2:2011-12 and DIN EN ISO 14419:2010 on an article which has been etched with an (APT) step and subsequently coated with a siloxane based coating (DHF). The measurement has been conducted before and after abrasion test. Table 9 shows a significant abrasion resistance of the coating. The contact angles with two liquids remain unchanged, indicating high coating adhesion to the object. However, we found rather poor oleophobic properties on the coated substrate due to droplet penetration through the very large mesh opening.

**Table 9. Comparative example contact angle with two liquids and oil drop test before and after abrasion test**

| Fabric | Treatment type | Contact angle [°] according to DIN 55660-2:2011-12 | | Oil drop test according to DIN EN ISO 14419:2010 [grade 0-8] |
|---|---|---|---|---|
| | | Water | Diiodomethane [°] | |
| G | APT+DHF before abrasion | 125 ±2 | 124 ±1 | 0 |
| | APT+DHF after abrasion | 128 ±3 | 122 ±2 | 0 |

### Example 9

In order to make sure that the DLC coating, when used as an undercoat for the DHF, can improve the chemical resistance of the resulted coating (DLC+DHF) compared to DHF only, an internal chemical dipping test has been performed at room temperature. The samples have been dipped in an acidic environment with around pH 1 and the dipping times are respectively 0, 15, 30, 60 and 90 min. The samples have been dried after the chemical treatment and the contact angle with two liquids has been measured according to DIN 55660-2:2011-12.

Table 10 reveals a considerable chemical resistance of DLC + DHF steps compared to DHF only. It can be clearly seen that higher contact angles were obtained on DLC+DHF coated and chemically treated samples compared to DHF coating, meaning an improved chemical resistance can be obtained with DLC step followed by DHF step.

**Table 10. Comparative example contact angle with two liquids before and after chemical test**

| Fabric | Treatment type | Dipping time in acetic acid [min] | Contact angle [°] according to DIN 55660-2:2011-12 | |
|---|---|---|---|---|
| | | | Water con- tact angle [°] | Diiodomethane contact angle [°] |
| C | DHF | 0 | 119 ±1 | 96 ±3 |
| | | 15 | 119 ±2 | 90 ±2 |
| | | 30 | 117 ±2 | 89 ±2 |
| | | 60 | 118 ±3 | 91 ±3 |
| | | 90 | 117 ±3 | 90 ±2 |
| | DLC+DHF | 0 | 127±2 | 114±1 |
| | | 15 | 120±1 | 112±2 |
| | | 30 | 120±2 | 110±1 |
| | | 60 | 119±2 | 108±2 |
| | | 90 | 118±1 | 107±2 |

### Example 10

An oil drop test has been measured respectively according to DIN EN ISO 14419:2010 on an article which has been coated one time with only a siloxane based coating (DHF) and another time with a DLC step followed by a DHF step. The measurements have been conducted before and after dipping test in alkaline as well as acidic medium with different treatment times as described in example 9.

As can be seen in table 11, unless we don't include a DLC step prior to a DHF step, the oil grade tends to drop significantly independent from the chemical nature of the medium (acid or alkali). This proves that the DLC step when performed before a DHF step can improve the chemical stability of the resulted coating.

**Table 11. Comparative example oil drop test before and after chemical test**

| Fabric | Treatment type | Dipping medium | Dipping time [min] | Oil crop test accord-ing to DIN EN ISO 14419:2010 [grade 0-8] |
|---|---|---|---|---|
| C | DHF | none | 0 | 3 |
| | | NaOH (0.5 g/l) / pH 10 | 15 | 2 |
| | | NaOH (0.5 g/l) / pH 10 | 30 | 2 |
| | | NaOH (0.5 g/l) / pH 10 | 60 | 1 |
| | | NaOH (0.5 g/l) / pH 10 | 90 | 1 |
| | DLC+DHF | none | 0 | 4 |
| | | NaOH (0.5 g/l) / pH 10 | 15 | 3 |
| | | NaOH (0.5 g/l) / pH 10 | 30 | 3 |
| | | NaOH (0.5 g/l) / pH 10 | 60 | 3 |
| | | NaOH (0.5 g/l) / pH 10 | 90 | 3 |
| | DHF | none | 0 | 3 |
| | | CH3COOH (300 g/l) / pH 1 | 15 | 2 |
| | | CH3COOH (300 g/l) / pH 1 | 30 | 1 |
| | | CH3COOH (300 g/l) / pH 1 | 60 | 1 |
| | | CH3COOH (300 g/l) / pH 1 | 90 | 1 |
| | DLC+DHF | none | 0 | 4 |
| | | CH3COOH (300 g/l) / pH 1 | 15 | 3 |
| | | CH3COOH (300 g/l) / pH 1 | 30 | 3 |
| | | CH3COOH (300 g/l) / pH 1 | 60 | 2 |
| | | CH3COOH (300 g/l) / pH 1 | 90 | 2 |

Based on the invention it is possible to provide a method of of producing a fabric having a halogen free plasma coating, especially free of per- and polyfluorinated substances (PFAS), according to standard IEC 62321-3-2:2020, EN 14582:2016 and/or ASTM D7359:2018, having a hydro- and oleophobic characteristics as well as a fabric comprising a halogen free plasma coating, especially free of per- and polyfluorinated substances (PFAS), having a hydro- and oleophobic characteristics.

## Claims

1. A method of producing a fabric having a halogen free plasma coating, especially free of per- and polyfluorinated substances (PFAS), according to standard IEC 62321-3-2:2020, EN 14582:2016 and/or ASTM D7359:2018, having a hydro- and oleophobic characteristics, wherein the method comprises:
step DHF of depositing a plasma coating on the fabric by means of plasma polymerization of a halogen free precursor monomer by plasma-enhanced chemical vapor deposition method (PECVD),
wherein the halogen free precursor monomer are organosilane, siloxane and/or hydrocarbon precursors,
wherein the plasma-enhanced chemical vapor deposition is carried out as a low-pressure plasma processes under protective atmosphere,
wherein the fabric comprises of a woven monofilament fabric of polymeric material having a filament diameter between 10 µm to 100 µm and a mesh opening between 5 µm and 200 µm.

2. Method according to claim 1,
**characterized in that**
comprising a step DLC of coating the fabric by means of sputtering of a carbon target by PVD process using an Argon plasma and/or using a hydrocarbon gas by PECVD method,
wherein the step DLC is carried out before step DHF.

3. Method according to claim 1 or 2,
**characterized in that**
comprising an APL step of creating an adhesion promoting layer by plasma
polymerization of hydrocarbon gas and/or a mixture of hydrocarbon, reactive and inert gases,
wherein the APL step is carried out before step DHF or before step DLC.

4. Method according to claim 1 or 2,
**characterized in that**
comprising an APT step of creating an adhesion promoting surface by using plasma etching, an ion beam irradiation and/or UV imprinting,
wherein the APT step is carried out before step DHF or before step DLC.

5. Method according to any one of the preceding claims,
**characterized in that**
in step DHF the halogen free hydro- and oleophobic coating is deposited on the fabric having a thickness from 30 nm to 300 nm.

6. Method according to any one of the preceding claims,
**characterized in that**
in step DLC the hydrophobic carbon coating is deposited on the fabric having a thickness from 5 nm to 200 nm.

7. Method according to any one of the preceding claims,
**characterized in that**
in step APL an adhesion promoting coating is deposited on the fabric having a thickness from 5 nm to 100 nm.

8. Method according to any one of the preceding claims,
**characterized in that**
wherein the fabric has a filament diameter between 10 µm to 100 µm, in particular preferably 19 µm to 50 µm.

9. Method according to any one of the preceding claims,
**characterized in that**
wherein the fabric has a mesh opening between 5 µm to 200 µm, in particular preferably 19 µm to 125 µm.

10. Method according to any one of the preceding claims,
**characterized in that**
wherein the fabric being plasma coated in step DHF has a hydrophobic character corresponding to water contact angle between 110° to 160° and oleophobic character corresponding to Diiodomethane contact angle between 80° to 140° and Hexadecane contact angle between 40° to 120° according to DIN 55660-2:2011-12 and oil grade up to 4 according to DIN EN ISO 14419:2010.

11. Method according to any one of the preceding claims,
**characterized in that**
wherein the fabric being coated in step DLC is chemically inert and resistant to acid, alkali and organic solvents and has a hydrophobic character corresponding to water contact angle between 90° to 140° according to DIN 55660-2:2011-12.

12. Method according to any one of the preceding claims,
**characterized by** further comprising
a step PT of a pre-treatment of the fabric by means of an atmospheric or low-pressure plasma using non-polymerforming an inert gas and/or a reactive gas wherein step PT is carried out before step DHF or DLC or APL, as first step.

13. Method according to any one of the preceding claims,
**characterized in that**
the steps are carried out in the following order:
1. PT,
2. APL or APT,
3. optionally DLC and
4. DHF.

14. Fabric comprising a halogen free plasma coating, especially free of per- and polyfluorinated substances (PFAS), according to standard IEC 62321-3-2:2020, EN 14582:2016 and/or ASTM D7359:2018 formed thereon having a hydro- and oleophobic characteristics by a method according to any one of the preceding claims,
wherein the fabric comprises of a woven monofilament fabric of polymeric material having a filament diameter between 10 µm to 100 µm and a mesh opening between 5 µm and 200 µm.
